# EUROPEAN PATENT SPECIFICATION

(11) **EP 1 909 637 B1**
(45) Date of publication and mention of the grant of the patent: **11.03.2009**
(21) Application number: 06788496.5
(22) Date of filing: 26.07.2006
(51) Int. Cl.: A61B 3/15, A61F 9/01

(54) **OPHTHALMIC DEVICE LATERAL POSITIONING SYSTEM AND ASSOCIATED METHODS**
SEITENPOSITIONIERUNGSSYSTEM EINER OPHTHALMISCHEN VORRICHTUNG UND ENTSPRECHENDE VERFAHREN
SYSTEME DE POSITIONNEMENT LATERAL D'UN DISPOSITIF OPHTALMIQUE ET PROCEDES ASSOCIES

(30) Priority: 29.07.2005 US 703669 P
(43) Date of publication of application: 16.04.2008
(73) Proprietor: Alcon RefractiveHorizons, Inc., Fort Worth, Texas 76134 (US)
(72) Inventor: CAMPIN, John, A., Orlando, FL 32828 (US); BOWES, John, J., Orlando, FL 32828 (US)
(74) Representative: Hanna, Peter William Derek
(86) International application number: PCT/US2006/028936
(87) International publication number: WO 2007/016132

(56) References cited:
- EP-A2- 0 765 648
- WO-A2-02/064031
- WO-A2-20/04089214
- US-B1- 6 257 722

## Description

### CROSS-REFERENCE TO RELATED APPLICATIONS

This application claims priority under 35 U.S.C. §119 to U.S. Provisional Patent Application No. 60/703,669, filed July 29, 2005.

### TECHNICAL FIELD OF THE INVENTION

The present invention relates to systems and methods for performing corneal wavefront measurements and laser-assisted corneal surgery, and, more particularly, to such systems and methods for optimizing a lateral positioning of the eye undergoing such surgery.

### BACKGROUND OF THE INVENTION

It is known in the art to perform corneal ablation by means of wavefront-guided refractive laser surgery. Typically a wavefront sensor measures the aberrations in an eye to produce an aberration map and determines its position relative to anatomical landmarks, which can be intrinsic or externally applied features. Aberration data, sometimes along with geometric registration information, can be transferred directly to a treatment excimer laser, which is typically used to perform the ablation.

In ophthalmic devices the positioning of a measuring or ablation device in a known position laterally relative to an eye such that the device can be therapeutically effective is of great importance. In some systems the eye must be centered and in clear focus for interaction of the image with an operator. It can also be important for a laser beam to come to focus at a predetermined plane with respect to the eye, for example, in an excimer laser system, or to have the eye positioned for an effective subsequent measurement of the eye, for example, a wavefront measurement.

Among the known techniques for assisting in positioning are the breaking of a plurality of light beams, such as infrared light beams, by the corneal apex, and the projection onto the cornea of a plurality of light beams, which can subsequently be analyzed either automatically or by an operator to assess accuracy of eye positioning. If the eye is deemed not to be in a therapeutically effective position, then the device and/or head/eye can be moved so as to reposition the eye optimally or to within defined acceptable tolerances.

Known current approaches to solving the positioning problem are typically subject to error and require intervention by an operator and/or additional hardware. Therefore, it would be advantageous to provide a system and method for improving accuracy and automation in eye alignment, without the need for human operator input or for additional hardware.

Document US 6,257,722 discloses a prior art device for determining the position of the center of the pupil relative to an ophthalmic device. Once an image of the eye is received by a computer, the light and dark information on the reflected light of the infrared light from the light sources are detected. In this detection, the image is divided into 16 areas. In view of the light and dark information of the pixels of every area, the computer determines the darkest area or area comprising the pupil. After the approximately whole pupil is detected, then, the pupil center position is detected. In order to do this, the distribution of light quantity information along an horizontal and a vertical line enables coordinates of the pupil edge position to be detected. The coordinates of the pupil edge position provide the center position of the coordinates, that is, coordinates of the pupil center position.

### BRIEF SUMMARY OF THE INVENTION

The invention is defined in the appended claims.

The present invention is directed to a system and method for determining a lateral position of an eye relative to an ophthalmic device. An optimal lateral position can be any position that places the eye such that the ophthalmic device can be therapeutically effective in its designed for purpose. Optimal lateral positioning can include positioning the eye such that the ophthalmic device can perform to the limits of its design tolerances, as well as anywhere in the ophthalmic devices designed for therapeutically effective range. An embodiment of the method of the present invention comprises the step of receiving data comprising an image of a surface of an eye. An edge feature in the image is located, wherein the edge feature is in a known relationship to a pupil of the eye. The image is mapped from the edge feature to laterally define the pupil, and a center of the pupil is determined using the pupil definition. The pupil center comprises a location from which to achieve an optimal lateral eye position relative to an ophthalmic device.

An embodiment of the system of the present invention can comprise a processor and a software package executable by the processor. The software package is adapted to carry out the above method steps.

Embodiments of the system and method of the present invention have an advantage that no additional hardware is required if the ophthalmic device already comprises means for imaging the surface of the eye and for capturing that image. An additional element can comprise a software package for computing optimal centering and focal position, and for either driving the ophthalmic device position or for indicating a required ophthalmic device movement, depending upon the presence of an automatic positioning capability.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### BRIEF DESCRIPTION OF THE SEVERAL VIEWS OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a simplified block diagram illustrating one embodiment of the eye lateral positioning system of the present invention;
FIGURE 2 is a flowchart of an exemplary embodiment of the method of the present invention;
FIGURE 3 is an image of an eye with the pupil de-centered; and
FIGURE 4 is an image of the eye with the pupil centered in accordance with the teachings of this invention.

### DETAILED DESCRIPTION OF THE PREFERRED EMBODIMENTS

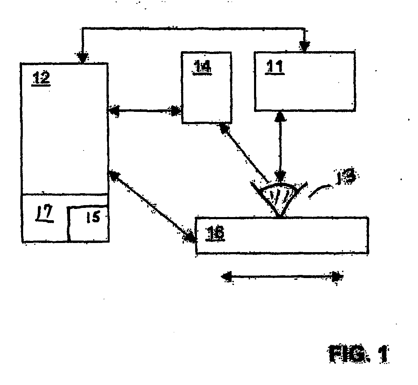

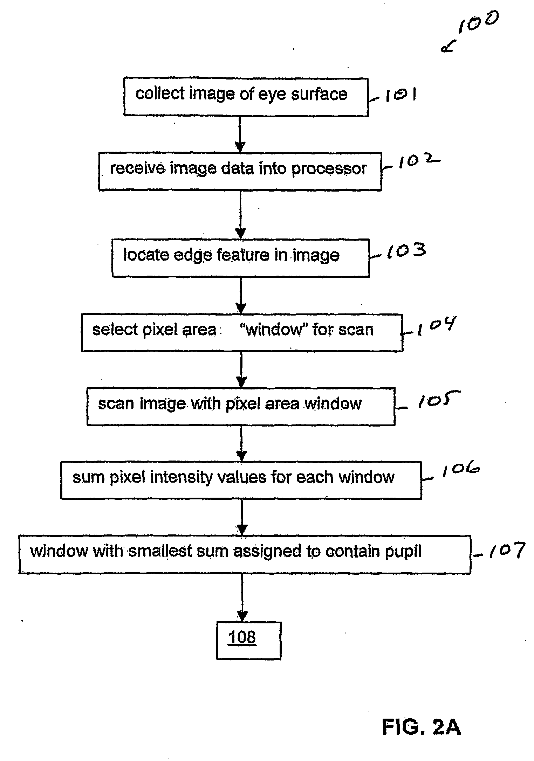

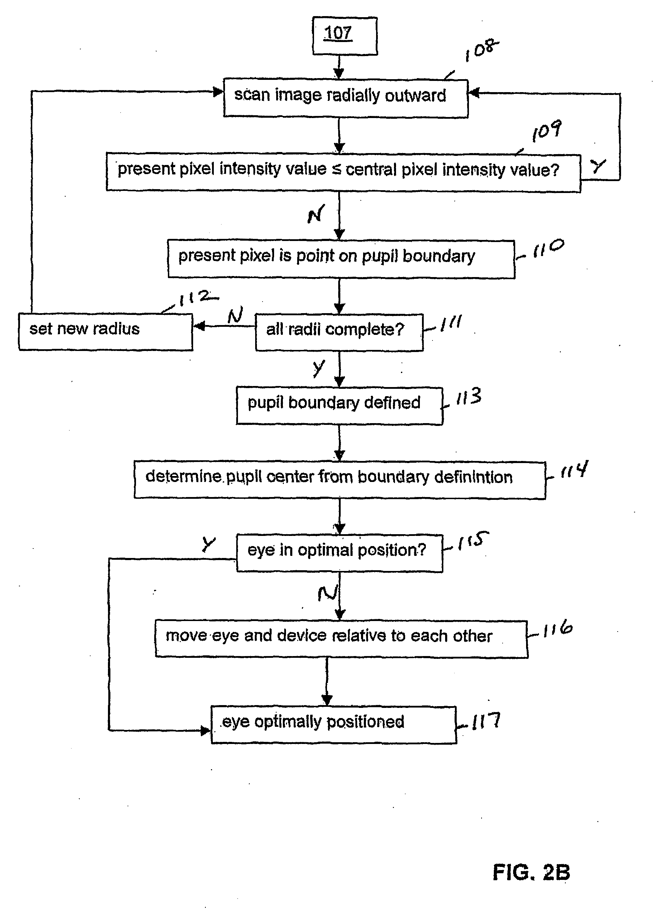

A description of the preferred embodiments of the present invention will now be presented with reference to FIGUREs 1-4. An exemplary eye positioning system **10** is depicted schematically in FIGURE 1, and an exemplary method **100,** in FIGUREs 2a and 2b.

An embodiment **100** of the method for determining an optimal position of an eye 13 relative to an ophthalmic device **11** comprises the step of receiving data into a processor **12** (block **102**). The data comprise an image of a surface of an eye **13** that has been collected (block **101**) with, for example, a video camera, digital camera, still camera or frame grabber **14,** in communication with the processor **12.** The image is collected with the eye at a first position relative to the ophthalmic device **11** (block **101**), and typically comprises a plurality of pixels, with each pixel having an intensity value associated therewith. Ophthalmic device **11** can be, for example, and without limitation, a femtosecond laser microkeratome, a treatment laser, such as an excimer laser, an aberrometer, or any other ophthalmic device, as will be known to those having skill in the art, for which accurate lateral positioning of an eye may be required.

A software package **15,** which can be resident in a memory **17** (here shown as part of processor **12**), includes a code segment for locating an edge feature in the image (block **103**). Memory **17** can be a separate memory operably coupled to processor **12,** or can be an integral part of processor **12.** The edge feature may include, but is not intended to be limited to, a pupil feature or a feature of the iris.

Processor **12** (control circuit) may be a single processing device or a plurality of processing devices. Such a processing device may be a microprocessor, microcontroller, digital signal processor, microcomputer, central processing unit, field programmable gate array, programmable logic device, state machine, logic circuitry, analog circuitry, digital circuitry, and/or any device that manipulates signals (analog and/or digital) based on operational instructions. The memory **17** coupled to the processor **12** or control circuit may be a single memory device or a plurality of memory devices. Such a memory device may be a read-only memory, random access memory, volatile memory, non-volatile memory, static memory, dynamic memory, flash memory, cache memory, and/or any device that stores digital information. Note that when the microprocessor or control circuit implements one or more of its functions via a state machine, analog circuitry, digital circuitry, and/or logic circuitry, the memory storing the corresponding operational instructions may be embedded within, or external to, the circuitry comprising the state machine, analog circuitry, digital circuitry, and/or logic circuitry. The memory stores, and the microprocessor or control circuit executes, operational instructions (e.g., software package **15**) corresponding to at least some of the steps and/or functions illustrated and described in association with FIGUREs 2A and 2B.

The image is mapped from the edge feature to laterally define the pupil, for example, by scanning from the edge feature to locate a darkest region in the image. This may be accomplished in an exemplary method by setting a rectangular area, or "window," that has a predefined size significantly smaller than a size of the image, but sufficiently large to contain a plurality of pixels (block **104**). This rectangular area is "slid" across the image, scanning every row until substantially the entire image has been scanned (block **105**). For each of the rectangular areas, the intensity values of each pixel within that area are summed (block **106**), yielding an intensity value for each of a plurality of regions within the image. A region having a smallest intensity value comprises a darkest region and is assigned to contain at least a portion of the pupil (block **107**).

Next, the image is scanned radially outward from a central pixel of the darkest region (block 108). The intensity value of each subsequent pixel is compared with the intensity value of the central pixel (block **109**). If the intensity value of the currently examined pixel is equal to or less than the central pixel's intensity value, the program continues to the next radially outward pixel (block **108**). If the intensity value of the currently examined pixel is greater than the central pixel's intensity value, the current pixel is considered to define a point on the pupil boundary (block **110**).

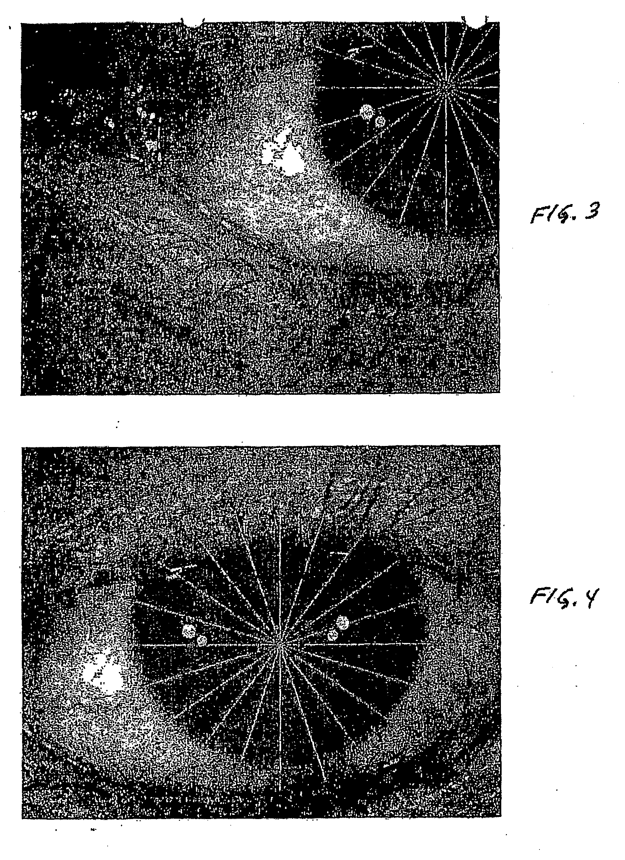

This procedure is repeated a predetermined number of times (block **111**) along different radii (block **112**), with the pupil boundary points collectively defining the pupil boundary (block **113**). A center of the pupil can then be determined from the boundary points (block **114**), as illustrated in FIGURE 3. The pupil center comprises a location from which to achieve an optimal lateral eye position relative to the ophthalmic device **11**. An optimal lateral position can be any position that places the eye such that the ophthalmic device **11** can be therapeutically effective in its designed for purpose. Optimal lateral positioning can include positioning the eye such that the ophthalmic device **11** can perform to the limits of its design tolerances, as well as anywhere in the ophthalmic devices designed for therapeutically effective range. An optimal lateral position can be a preferred lateral position of an eye relative to an ophthalmic device.

If the eye is in a position other than an optimal lateral position (block **115**), as determined from the determined pupil center and the intended ophthalmic device **11** operating parameters, the eye and the ophthalmic device **11** are relatively repositioned (block **116**) to place the eye in the optimal lateral position (block **117**), as illustrated in FIGURE 4. Such repositioning may be effected manually or automatically under control of the software **15** and processor **12,** by means which will be familiar to those having skill in the art and which are intended to be within the scope of the present invention, such as by using a positioning device **16.** For example, and without limitation, the patient can be manually repositioned, the ophthalmic device **11** can be manually repositioned, and/or the ophthalmic device **11** or table/chair (e.g., positioning device **16**) on which the patient is being supported can be automatically repositioned by mechanical and electrical control systems, or any combination of these methods. Once the eye is in the desired position, a desired procedure can be performed on the eye **13** using the ophthalmic device **11.** The embodiments of this invention thus provide a pupil center reference point from which an optimal positioning of an eye and a treating ophthalmic device **11** can be determined.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

## Claims

1. A method for determining a preferred lateral position of an eye (13) relative to an ophthalmic device (11), comprising the steps of:
receiving (102) data comprising an image of a surface of an eye, the image comprising a plurality of pixels;
locating an edge feature in the image (103), the edge feature in a known relationship to a pupil of the eye;
mapping the image from the edge feature to laterally define the pupil by scanning (105) from the edge feature to locate a darkest region in the image, by calculating (106) an intensity value for each of a plurality of regions within the image, each region having a predefined size significantly smaller than a size of the image, a region having a smallest intensity value comprising a darkest region and assigned (107) to contain at least a portion of the pupil, the region size sufficiently large to contain a plurality of pixels;
defining a boundary of the darkest region;
scanning the image radially outward (108) from a central pixel of the darkest region, the central pixel having a first intensity value, and determining (109) a pixel closest to the central pixel in the outward scan having a second intensity value greater than the first intensity value; and
determining a center of the pupil (114) using the defined pupil map by calculating a geometric center of the darkest region, the pupil center comprising a location from which to achieve a preferred lateral eye position (117) relative to an ophthalmic device.

2. The method recited in Claim 1, wherein the edge feature is selected from a group consisting of a pupil feature and an iris feature.

3. The method recited in Claim 1, further comprising repeating the radial scanning and pixel determining steps (108,109) along a plurality of different radii (112) to define a pupil boundary (113).

4. The method recited in Claim 1, further comprising the step of, if the eye is in a position other than the preferred lateral position, relatively repositioning (116) the eye and the ophthalmic device to place the eye in the preferred lateral position (117).

5. A system for determining a preferred lateral position of an eye (13) relative to an ophthalmic device (11) comprising:
a processor (12); and
a software package (15) installable on the processor adapted to:
receive (102) data comprising an image of a surface of an eye, the image comprising a plurality of pixels;
locate an edge feature in the image (103), the edge feature in a known relationship to a pupil of the eye;
map the image from the edge feature to laterally define the pupil by scanning (105) from the edge feature to locate a darkest region in the image, by calculating (106) an intensity value for each of a plurality of regions within the image, each region having a predefined size significantly smaller than a size of the image, a region having a smallest intensity value comprising a darkest region and assigned (107) to contain at least a portion of the pupil, the region size sufficiently large to contain a plurality of pixels;
define a boundary of the darkest region;
scan the image radially outward (108) from a central pixel of the darkest region, the central pixel having a first intensity value, and determining (109) a pixel closest to the central pixel in the outward scan having a second intensity value greater than the first intensity value; and
determine a center of the pupil (114) using the defined pupil map by calculating a geometric center of the darkest region, the pupil center comprising a location from which to achieve a preferred lateral eye position (117) relative to an ophthalmic device.

6. The system recited in Claim 5, wherein the edge feature is selected from a group consisting of a scleral blood vessel and an iris feature.

7. The system recited in Claim 5, wherein the software package (15) is further adapted to repeat the radial scanning and pixel determining (108,109) along a plurality of different radii (112) to define a pupil boundary (113).

8. The system recited in Claim 7, further comprising, if the eye is in a position other than the preferred lateral position, means for relatively repositioning (116) the eye and the ophthalmic device to place the eye in the preferred lateral position (117).

9. Software (15) which when executing on a computer processor (12) performs the steps of any one of claims 1 to 4.

## Patentansprüche

1. Verfahren zur Bestimmung der bevorzugten lateralen Position eines Auges (13) relativ zu einem ophthalmischen Gerät (11), mit den Schritten:
Empfangen (102) von Daten, die das Bild der Oberfläche eines Auges aufweisen, wobei das Bild eine Vielzahl Bildpunkte aufweist;
Lokalisieren eines Randmerkmals im Bild (103), wobei das Randmerkmal eine bekannte Beziehung zur Pupille des Auges hat;
Abbilden des Bildes vom Randmerkmal aus durch Abtasten (105) vom Randmerkmal aus, um die Pupille lateral zu definieren, um die dunkelste Zone im Bild zu lokalisieren, indem für jede der Mehrzahl Zonen im Bild ein Intensitätswert berechnet wird (106), wobei jede Zone eine vorgegebene Größe hat, die deutlich kleiner ist als die Größe des Bildes, wobei eine Zone mit dem niedrigsten Intensitätswert, die die dunkelste Zone aufweist, als zumindest einen Abschnitt der Pupille enthaltend festgesetzt (107) wird und die Zone hinreichend groß ist, um eine Vielzahl Bildpunkte zu enthalten;
Definieren der Grenze der dunkelsten Zone;
Abtasten des Bildes von einem zentralen Bildpunkt der dunkelsten Zone aus radial nach außen (108), wobei der zentrale Bildpunkt einen ersten Intensitätswert hat, und Bestimmen (109) eines Bildpunktes, der dem zentralen Bildpunkt bei der Auswärtsabtastung am nächsten liegt und einen zweiten Intensitätswert hat, der größer ist als der erste Intensitätswert; und
Bestimmen des Mittelpunktes der Pupille (114) mittels der definierten Pupillenabbildung, indem der geometrische Mittelpunkt der dunkelsten Zone berechnet wird, wobei der Pupillenmittelpunkt einen Ort aufweist, von dem aus die bevorzugte laterale Augenposition (117) relativ zu einem ophthalmischen Gerät erhalten werden kann.

2. Verfahren nach Anspruch 1, bei dem das Randmerkmal aus einer Gruppe ausgewählt wird, die aus einem Pupillen- und einem Irismerkmal besteht.

3. Verfahren nach Anspruch 1, das ferner die Wiederholung der Schritte der radialen Abtastung und der Bestimmung der Bildpunkte (108, 109) entlang einer Mehrzahl verschiedener Radien (112) aufweist, um die Pupillengrenze zu bestimmen (113).

4. Verfahren nach Anspruch 1, das dann, wenn sich das Auge in einer anderen als der bevorzugten lateralen Position befindet, ferner den Schritt der relativen Repositionierung (116) des Auges und des ophthalmischen Geräts aufweist, um das Auge in die bevorzugte laterale Position zu bringen (117).

5. System zur Bestimmung der bevorzugten lateralen Position eines Auges (13) relativ zu einem ophthalmischen Gerät (11), mit:
einem Prozessor (12); und
einem Software-Paket (15), das im Prozessor installierbar und eingerichtet ist, um:
Daten zu empfangen (102), die das Bild der Oberfläche eines Auges aufweisen, wobei das Bild eine Vielzahl Bildpunkte aufweist;
ein Randmerkmal im Bild zu lokalisieren (103), wobei das Randmerkmal eine bekannte Beziehung zur Pupille des Auges hat;
das Bild vom Randmerkmal aus durch Abtasten (105) vom Randmerkmal aus abzubilden, um die Pupille lateral zu definieren, um die dunkelste Zone im Bild zu lokalisieren, indem für jede der Mehrzahl Zonen im Bild ein Intensitätswert berechnet wird (106), wobei jede Zone eine vorgegebene Größe hat, die deutlich kleiner ist als die Größe des Bildes, wobei eine Zone mit dem niedrigsten Intensitätswert, die die dunkelste Zone aufweist, als zumindest einen Abschnitt der Pupille enthaltend festgesetzt (107) wird und die Zone hinreichend groß ist, um eine Vielzahl Bildpunkte zu enthalten;
die Grenze der dunkelsten Zone zu definieren;
das Bild von einem zentralen Bildpunkt der dunkelsten Zone aus radial nach außen (108) abzutasten, wobei der zentrale Bildpunkt einen ersten Intensitätswert hat, und einen Bildpunkt zu bestimmen (109), der dem zentralen Bildpunkt bei der Auswärtsabtastung am nächsten liegt und einen zweiten Intensitätswert hat, der größer ist als der erste Intensitätswert; und
den Mittelpunkt der Pupille (114) mittels der definierten Pupillenabbildung zu bestimmen, indem der geometrische Mittelpunkt der dunkelsten Zone berechnet wird, wobei der Pupillenmittelpunkt einen Ort aufweist, von dem aus die bevorzugte laterale Augenposition (117) relativ zu einem ophthalmischen Gerät erhalten werden kann.

6. System nach Anspruch 5, bei dem das Randmerkmal aus einer Gruppe ausgewählt wird, die aus einem skleralen Blutgefäß und einem Irismerkmal besteht.

7. System nach Anspruch 5, bei dem das Software-Paket (15) ferner dazu eingerichtet ist, die radiale Abtastung und die Bestimmung der Bildpunkte (108, 109) entlang einer Mehrzahl verschiedener Radien (112) zu wiederholen, um die Pupillengrenze zu bestimmen (113).

8. System nach Anspruch 7, das dann, wenn sich das Auge in einer anderen als der bevorzugten lateralen Position befindet, Mittel zur relativen Repositionierung (116) des Auges und des ophthalmischen Geräts aufweist, um das Auge in die bevorzugte laterale Position zu bringen (117).

9. Software (15), die bei Ausführung auf einem Computerprozessor (12) die Schritte der Ansprüche 1 bis 4 ausführt.

## Revendications

1. Procédé de détermination d'une position latérale préférée d'un oeil (13) par rapport à un dispositif ophtalmique (11), comportant les étapes consistant à :
recevoir (102) des données comportant une image d'une surface d'un oeil, l'image comportant une pluralité de pixels,
localiser une caractéristique de bord sur l'image (103), la caractéristique de bord étant dans une relation connue par rapport à une pupille de l'oeil,
cartographier l'image à partir de la caractéristique de bord afin de définir latéralement la pupille par balayage (105) à partir de la caractéristique de bord afin de localiser la région la plus sombre dans l'image, en calculant (106) une valeur d'intensité pour chacune d'une pluralité de régions dans l'image, chaque région ayant une dimension prédéfinie sensiblement plus petite qu'une taille de l'image, une région ayant la valeur d'intensité la plus faible comportant la région la plus sombre, et étant désignée (107) pour contenir au moins une partie de la pupille, la taille de la région étant suffisamment grande pour contenir une pluralité de pixels,
définir une limite de la région la plus sombre,
balayer l'image radialement vers l'extérieur (108) à partir d'un pixel central de la région la plus sombre, le pixel central ayant une première valeur d'intensité, et déterminer (109) un pixel le plus proche du pixel central dans le balayage vers l'extérieur ayant une seconde valeur d'intensité supérieure à la première valeur d'intensité, et
déterminer un centre de la pupille (114) en utilisant la cartographie pupillaire définie par calcul d'un centre géométrique de la région la plus sombre, le centre pupillaire comportant un emplacement à partir duquel il est possible d'atteindre une position oculaire latérale préférée (117) par rapport à un dispositif ophtalmique.

2. Procédé selon la revendication 1, dans lequel la caractéristique de bord est choisie dans un groupe constitué d'une caractéristique de pupille et d'une caractéristique d'iris.

3. Procédé selon la revendication 1, comportant en outre le fait de répéter les étapes de balayage radial et de détermination de pixel (108, 109) le long d'une pluralité de rayons différents (112) afin de définir une limite de pupille (113).

4. Procédé selon la revendication 1, comportant en outre l'étape consistant à, si l'oeil est dans une position autre que la position latérale préférée, repositionner de manière relative (116) l'oeil et le dispositif ophtalmique afin de placer l'oeil dans la position latérale préférée (117).

5. Système de détermination d'une position latérale préférée d'un oeil (13) par rapport à un dispositif ophtalmique (11), comportant :
un processeur (12), et
un progiciel (15) pouvant être installé sur le processeur conçu pour :
recevoir (102) des données comportant une image d'une surface d'un oeil, l'image comportant une pluralité de pixels,
localiser une caractéristique de bord dans l'image (103), la caractéristique de bord étant dans une relation connue par rapport à une pupille de l'oeil,
cartographier l'image à partir de la caractéristique de bord afin de définir latéralement la pupille par balayage (105) à partir de la caractéristique de bord pour localiser la région la plus sombre dans l'image, en calculant (106) une valeur d'intensité pour chacune d'une pluralité de régions dans l'image, chaque région ayant une taille prédéfinie sensiblement plus petite qu'une taille de l'image, une région ayant la valeur d'intensité la plus petite comportant la région la plus sombre, étant désignée (107) pour contenir au moins une partie de la pupille, la taille de la région étant suffisamment grande pour contenir plusieurs pixels,
définir une limite de la région la plus sombre,
balayer l'image radialement vers l'extérieur (108) à partir d'un pixel central de la région la plus sombre, le pixel central ayant une première valeur d'intensité, et déterminer (109) un pixel le plus proche du pixel central dans le balayage vers l'extérieur ayant une seconde valeur d'intensité supérieure à la première valeur d'intensité, et
déterminer un centre de la pupille (114) en utilisant la cartographie de pupille définie par calcul d'un centre géométrique de la région la plus foncée, le centre de pupille comportant un emplacement à partir duquel il est possible d'atteindre une position oculaire latérale préférée (117) par rapport à un dispositif ophtalmique.

6. Système selon la revendication 5, dans lequel la caractéristique de bord est choisie dans un groupe constitué d'un vaisseau sanguin scléral et une caractéristique d'iris.

7. Système selon la revendication 5, dans lequel le progiciel (15) est en outre conçu pour répéter le balayage radial et la détermination de pixel (108, 109) le long d'une pluralité de rayons différents (112) afin de définir une limite pupillaire (113).

8. Système selon la revendication 7, comportant en outre, si l'oeil est dans une position autre que la position latérale préférée, des moyens pour repositionner de manière relative (116) l'oeil et le dispositif ophtalmique afin de placer l'oeil dans la position latérale préférée (117).

9. Logiciel (15) qui, lors de l'exécution sur un processeur informatique (12), réalise les étapes de l'une quelconque des revendications 1 à 4.
